# EUROPEAN PATENT APPLICATION

(11) **EP 3 058 863 A1**
(43) Date of publication of application: **24.08.2016**
(21) Application number: 14854616.1
(22) Date of filing: 22.07.2014
(51) Int. Cl.: A61B 1/04, A61B 1/00

(54) **EXTRACORPOREAL TERMINAL, CAPSULE ENDOSCOPE SYSTEM, CAPSULE-ENDOSCOPE CONTROL METHOD, AND PROGRAM**

(30) Priority: 16.10.2013 JP 2013215738
(71) Applicant: Olympus Corporation, Hachioji-shi Tokyo 192-8507 (JP)
(72) Inventor: HASEGAWA, Yasuhiro, Tokyo 192-8507 (JP)
(74) Representative: Gunzelmann, Rainer
(86) International application number: PCT/JP2014/069366
(87) International publication number: WO 2015/056475

(57) **Abstract**

An extracorporeal terminal includes a wireless communication unit including a plurality of antenna elements, and configured to transmit and receive a signal with a capsule endoscope that is able to set a frame rate, a storage unit configured to store a position database showing relationships between coordinates and the frame rate, a position estimation unit configured to estimate a position of the capsule endoscope using a signal level of the signal received by the wireless communication unit, and a frame rate-setting unit configured to set the frame rate corresponding to the coordinates when a distance between a position shown by the coordinates included in the position database retained in the storage unit and a position of the capsule endoscope estimated by the position estimation unit is less than a distance threshold that is preset. The wireless communication unit transmits a signal showing the frame rate set by the frame rate-setting unit to the capsule endoscope.

## Description

### [Technical Field]

The present invention relates to an extracorporeal terminal, a capsule endoscope system, a capsule-endoscope control method and a program.

Priority is claimed on Japanese Patent Application No. 2013-215738, filed October 16, 2013, the content of which is incorporated herein by reference.

### [Background Art]

In the related art, endoscopes are widely used as medical observation devices configured to be introduced into the body of examinees such as patients or the like to observe the inside of body cavities. In addition, in recent years, a type of swallowable endoscope (a capsule endoscope) including a photographing device, a communication device configured to wirelessly transmit image data captured by the photographing device to the outside of the body, or the like, installed in a capsule type housing has been developed. The capsule endoscope has a function of moving through the inside of internal organs such as the esophagus, the stomach, the small intestine, the large intestine, or the like, according to peristaltic movement thereof, after swallowing, from the mouth of a patient to observe the inside of the body cavity until the endoscope is naturally discharged from the human body, and sequentially photographing the internal organs.

During the movement of the capsule endoscope through the body cavity, the image data captured in the body cavity by the capsule endoscope are sequentially transmitted to the outside of the body through wireless communication and accumulated in a memory provided at the inside or outside of an extracorporeal terminal or displayed on a display installed at the extracorporeal terminal as an image. The image data accumulated in the memory is input into an information-processing device via a cradle into which the extracorporeal terminal is inserted, and displays the image on a display of an information-processing device or displays the image on the display installed at the extracorporeal terminal while receiving the data. A doctor or a nurse can perform diagnosis based on the image.

In addition, since a moving speed of the capsule endoscope varies, photographing at an appropriate frame rate is needed. With respect to such requirements, a method of estimating movement information (a moving speed) of a capsule from differential information of a plurality of captured images or acceleration information acquired by an acceleration sensor installed at the capsule endoscope and controlling a frame rate of the capsule endoscope using the extracorporeal terminal is known (for example, see Patent Literature 1).

### [Citation List]

### [Patent Literature]

[Patent Literature 1] Japanese Patent No. 4864534

### [Summary of Invention]

### [Technical Problem]

However, in the technology disclosed in Patent Literature 1, since the capsule endoscope performs image capturing during passage through a portion other than that serving as an observation target, excessive consumption of a battery occurs.

In consideration of the above-mentioned circumstances, the present invention is directed to provide an extracorporeal terminal, a capsule endoscope system, a capsule-endoscope control method and a program that are capable of performing setting of a frame rate while suppressing an increase in electric power consumption of a capsule endoscope.

### [Solution to Problem]

According to a first aspect of the present invention, an extracorporeal terminal includes a wireless communication unit including a plurality of antenna elements and configured to transmit and receive a signal with a capsule endoscope that is capable of being set with a frame rate; a storage unit configured to retain a position database showing relationships between coordinates and the frame rate; a position estimation unit configured to estimate a position of the capsule endoscope using a signal level of the signal received by the wireless communication unit; and a frame rate-setting unit configured to set the frame rate corresponding to the coordinates when a distance between a position shown by the coordinates included in the position database retained in the storage unit and a position of the capsule endoscope estimated by the position estimation unit is less than a distance threshold that is preset. The wireless communication unit transmits a signal showing the frame rate set by the frame rate-setting unit to the capsule endoscope.

According to a second aspect of the present invention, in the extracorporeal terminal according to the first aspect, the extracorporeal terminal may further include a distance threshold modification unit configured to modify the distance threshold.

According to a third aspect of the present invention, in the extracorporeal terminal according to the second aspect, the extracorporeal terminal may further include a speed calculation unit configured to calculate a moving speed of the capsule endoscope based on an estimation result of the position estimation unit. The distance threshold modification unit may set a value of the distance threshold to a large value when the moving speed calculated by the speed calculation unit is larger than a first speed and sets a value of the distance threshold to a small value when the moving speed is smaller than a second speed.

According to a fourth aspect of the present invention, in the extracorporeal terminal according to the second aspect, the extracorporeal terminal may further include an image storage unit configured to store image data received by the wireless communication unit and captured by the capsule endoscope; and an image variation calculation unit configured to calculate an image variation serving as a chronological differential quantity of the image data stored in the image storage unit. The distance threshold modification unit may set a value of the distance threshold to a large value when the image variation calculated by the image variation calculation unit is larger than a first variation, and set a value of the distance threshold to a small value when the image variation is smaller than a second variation.

According to a fifth aspect of the present invention, in the extracorporeal terminal according to the second aspect, the extracorporeal terminal may further include a speed calculation unit configured to calculate a moving speed of the capsule endoscope based on an estimation result of the position estimation unit. The distance threshold modification unit may set a value obtained by adding a previously set threshold addition quantity to the distance threshold as a distance threshold when the moving speed calculated by the speed calculation unit is larger than a predetermined speed.

According to a sixth aspect of the present invention, in the extracorporeal terminal according to the second aspect, the extracorporeal terminal may further include an image storage unit configured to store image data received by the wireless communication unit and captured by the capsule endoscope; and an image variation calculation unit configured to calculate an image variation serving as a chronological differential quantity of the image data stored in the image storage unit. The distance threshold modification unit may set a value obtained by adding a preset threshold addition quantity to the distance threshold as a distance threshold when the image variation calculated by the image variation calculation unit is larger than a predetermined variation.

According to a seventh aspect of the present invention, in the extracorporeal terminal according to the first aspect, the extracorporeal terminal may further include a position database selection unit configured to select one of the relationships between the coordinates and the frame rates included in the position database retained in the storage unit; and a position database-setting unit configured to set the coordinates selected by the position database selection unit to coordinates showing the position estimated by the position estimation unit.

According to an eighth aspect of the present invention, in a capsule endoscope system including a capsule endoscope and an extracorporeal terminal, the capsule endoscope includes a capsule communication unit configured to transmit and receive a signal with the extracorporeal terminal; and an imaging unit configured to capture an image based on a signal showing a frame rate transmitted from the extracorporeal terminal. The extracorporeal terminal includes a wireless communication unit including a plurality of antenna elements and configured to transmit and receive the signal with the capsule endoscope; a storage unit configured to retain a position database showing relationships between coordinates and frame rates; a position estimation unit configured to estimate a position of the capsule endoscope using a signal level of the signal received by the wireless communication unit; and a frame rate-setting unit configured to set the frame rate corresponding to the coordinates when a distance between a position shown by the coordinates included in the position database retained in the storage unit and a position of the capsule endoscope estimated by the position estimation unit is less than a previously set distance threshold. The wireless communication unit transmits the signal showing the frame rate set by the frame rate-setting unit to the capsule endoscope.

According to a ninth aspect of the present invention, a capsule-endoscope control method includes a wireless communication step in which a wireless communication unit including a plurality of antenna elements transmits and receives a signal with a capsule endoscope that is capable of being set with a frame rate; a position estimation step of estimating a position of the capsule endoscope using a signal level of the signal received by the wireless communication unit; and a frame rate-setting step of setting a frame rate corresponding to coordinates when a distance between a position shown by coordinates included in a position database showing a relationship between coordinates and frame rates retained in a storage unit and the position of the capsule endoscope estimated in the position estimation step is less than a preset distance threshold. In the wireless communication step, a signal showing the frame rate set in the frame rate-setting step is transmitted to the capsule endoscope.

According to a tenth aspect of the present invention, a program causes a computer to execute a wireless communication step in which a wireless communication unit including a plurality of antenna elements transmits and receives a signal with a capsule endoscope that is capable of being set with a frame rate; a position estimation step of estimating a position of the capsule endoscope using a signal level of the signal received by the wireless communication unit; and a frame rate-setting step of setting a frame rate corresponding to coordinates when a distance between a position shown by coordinates included in a position database showing a relationship between coordinates and frame rates retained in a storage unit and the position of the capsule endoscope estimated in the position estimation step is less than a preset distance threshold. In the program, in the wireless communication step, a process of transmitting a signal showing the frame rate set in the frame rate-setting step to the capsule endoscope is executed in the computer.

### [Advantageous effects of Invention]

According to the extracorporeal terminal, the capsule endoscope system, the capsule-endoscope control method and the program of the present invention, the setting of the frame rate can be performed while suppressing an increase of electric power consumption of the capsule endoscope.

### [Brief Description of Drawings]

Fig. 1 is a block diagram showing a configuration of a capsule endoscope system according to a first embodiment of the present invention.
Fig. 2 is a block diagram showing a configuration of the capsule endoscope according to the first embodiment of the present invention.
Fig. 3 is a block diagram showing a configuration of an extracorporeal terminal according to the first embodiment of the present invention.
Fig. 4 is a schematic view showing a data structure of a position database according to the first embodiment of the present invention.
Fig. 5 is a schematic view showing a relationship between coordinates according to the first embodiment of the present invention and the human body.
Fig. 6 is a flow chart showing an operational sequence in which the extracorporeal terminal according to the first embodiment of the present invention determines a frame rate of the capsule endoscope and transmits information showing the determined frame rate to the capsule endoscope.
Fig. 7 is a block diagram showing a configuration of an extracorporeal terminal according to a second embodiment of the present invention.
Fig. 8 is a schematic view showing a data structure of a position database according to the second embodiment of the present invention.
Fig. 9 is a flow chart showing an operational sequence in which the extracorporeal terminal according to the second embodiment of the present invention determines a frame rate of the capsule endoscope and transmits information showing the determined frame rate to the capsule endoscope.
Fig. 10 is a block diagram showing a configuration of an extracorporeal terminal according to a third embodiment of the present invention.
Fig. 11 is a schematic view showing a data structure of a distance threshold table according to the third embodiment of the present invention.
Fig. 12 is a flow chart showing an operational sequence in which the extracorporeal terminal according to the third embodiment of the present invention determines a frame rate of the capsule endoscope and transmits information showing the determined frame rate to the capsule endoscope.
Fig. 13 is a block diagram showing a configuration of an extracorporeal terminal according to a fourth embodiment of the present invention.
Fig. 14 is a schematic view showing a data structure of a distance threshold table according to the fourth embodiment of the present invention.
Fig. 15 is a flow chart showing an operational sequence in which the extracorporeal terminal according to the fourth embodiment of the present invention determines a frame rate of the capsule endoscope and transmits information showing the determined frame rate to the capsule endoscope.
Fig. 16 is a schematic view showing a data structure of a distance threshold addition table according to a fifth embodiment of the present invention.
Fig. 17 is a flow chart showing an operational sequence in which an extracorporeal terminal according to the fifth embodiment of the present invention determines a frame rate of the capsule endoscope and transmits information showing the determined frame rate to the capsule endoscope.
Fig. 18 is a flow chart showing an operational sequence in which an extracorporeal terminal according to a sixth embodiment of the present invention determines a frame rate of a capsule endoscope and transmits information showing the determined frame rate to the capsule endoscope.
Fig. 19 is a schematic view showing a data structure of a distance threshold addition table according to the sixth embodiment of the present invention.
Fig. 20 is a block diagram showing a configuration of an extracorporeal terminal according to a seventh embodiment of the present invention.
Fig. 21 is a flow chart showing an operational sequence in which the extracorporeal terminal according to the seventh embodiment of the present invention updates a value of a data item "coordinates" of a position database.
Fig. 22 is a schematic view showing a relationship between the human body and a coordinate according to the seventh embodiment of the present invention.

### [Description of Embodiments]

### (First embodiment)

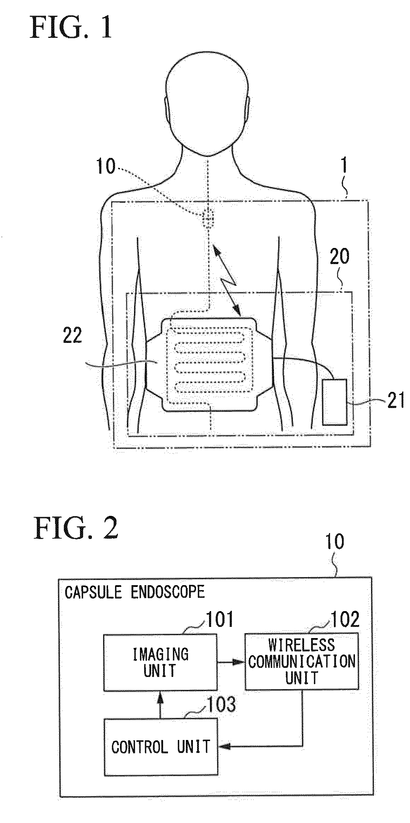

Hereinafter, a first embodiment of the present invention will be described with reference to the accompanying drawings. Fig. 1 is a block diagram showing a configuration of a capsule endoscope system according to the embodiment. In the example shown, a capsule endoscope system 1 includes a capsule endoscope 10 and an extracorporeal terminal 20. The extracorporeal terminal 20 includes a main body section 21 and an antenna section 22. The capsule endoscope 10 and the extracorporeal terminal 20 enable transmission and reception of data using wireless communication. The antenna section 22 includes a plurality of antennae to enable communication even when the capsule endoscope 10 is present at a specified position in a body cavity.

For example, the capsule endoscope 10 moves through a stomach, a small intestine or a large intestine according to peristaltic movement after the capsule endoscope 10 is swallowed from the mouth of a patient to observe the inside of the body cavity. In addition, for example, the capsule endoscope 10 captures images in the body cavity when the capsule endoscope 10 is present in the body cavity and sequentially transmits the captured image data to the extracorporeal terminal 20 using wireless communication. The extracorporeal terminal 20 stores the image data transmitted from the capsule endoscope 10. The extracorporeal terminal 20 may use the stored data for diagnosis or the like.

Fig. 2 is a block diagram showing a configuration of the capsule endoscope 10 according to the embodiment. In the example shown, the capsule endoscope 10 includes an imaging unit 101, a wireless communication unit 102 (a capsule communication unit) and a control unit 103. The imaging unit 101 performs imaging at a designated frame rate according to control of the control unit 103 and acquires image data. The wireless communication unit 102 includes antenna elements and performs wireless communication with the extracorporeal terminal 20. For example, the wireless communication unit 102 modulates the image data acquired by the imaging unit 101 to transmit the image data to the extracorporeal terminal 20. In addition, for example, the wireless communication unit 102 receives information showing a frame rate transmitted from the extracorporeal terminal 20. The control unit 103 performs control of parts included in the capsule endoscope 10. In addition, the control unit 103 controls the frame rate of the imaging unit 101 according to the information showing the frame rate received from the extracorporeal terminal 20 by the wireless communication unit 102.

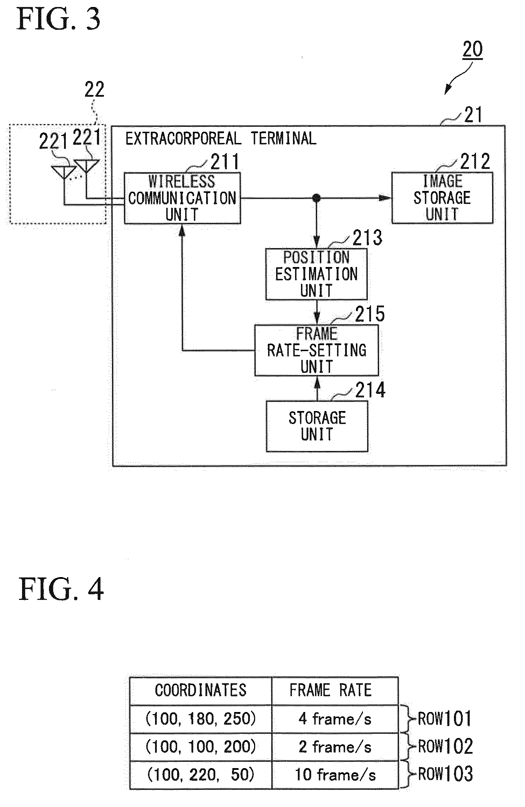

Fig. 3 is a block diagram showing a configuration of the extracorporeal terminal 20 according to the embodiment. In the example shown, the extracorporeal terminal 20 includes the main body section 21 and the antenna section 22. The main body section 21 includes a wireless communication unit 211, an image storage unit 212, a position estimation unit 213, a storage unit 214 and a frame rate-setting unit 215. The antenna section 22 includes a plurality of antenna elements 221. When the antenna section 22 is attached to the human body, a location at which the plurality of antenna elements 221 are disposed is predetermined. For example, the wireless communication unit 211 and the antenna section 22 correspond to a wireless communication unit in the accompanying claims.

The plurality of antenna elements 221 receive signals (modulated signals or non-modulated carrier waves) transmitted from the capsule endoscope 10. The wireless communication unit 211 demodulates the image data based on the signals received by the plurality of antenna elements 221. In addition, the wireless communication unit 211 measures a signal level (a received signal intensity) of the signals received by the plurality of antenna elements 221. In addition, the wireless communication unit 211 transmits the information showing the frame rate determined by the frame rate-setting unit 215 to the capsule endoscope 10 via the antenna elements 221.

The image storage unit 212, for example, a memory or the like, stores the image data modulated by the wireless communication unit 211. The position estimation unit 213 performs position estimation of the capsule endoscope 10 using the signal level of the signals received by the plurality of antenna elements 221 measured by the wireless communication unit 211. The position estimation method will be described below.

The storage unit 214 is, for example, a memory or the like, and stores a position database. The position database is a database configured to store coordinates for modifying the frame rate of the capsule endoscope 10 and a data set of the frame rate set upon arrival at the positions thereof. Details of the position database will be described below. The frame rate-setting unit 215 determines the frame rate of the imaging unit 101 of the capsule endoscope 10 based on the position database stored in the storage unit 214 and the position of the capsule endoscope 10 estimated by the position estimation unit 213. A method of determining the frame rate will be described below.

Next, the position database will be described. Fig. 4 is a schematic view showing a data structure of the position database of the embodiment. The position database has data items such as "coordinates" and "frame rate," and stores data of the data items in association with each row. The data item "coordinates" stores coordinates showing a position at which the frame rate of the imaging unit 101 of the capsule endoscope 10 is set. The data item "frame rate" stores the frame rate of the imaging unit 101 of the capsule endoscope 10.

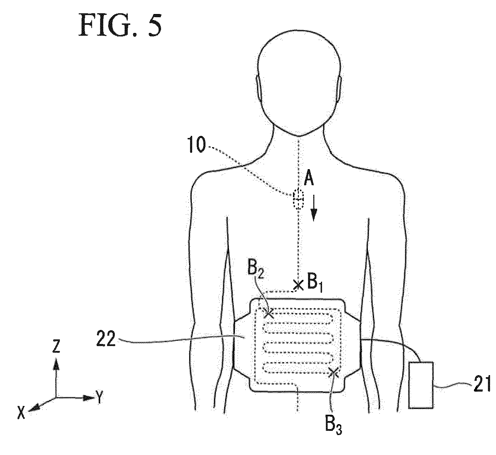

Fig. 5 is a schematic view showing a relationship between the coordinates of the embodiment and the human body. In the embodiment, an x-axis direction, a y-axis direction and a z-axis direction of the coordinates are directions shown in Fig. 5. As shown, the x-axis direction is a direction that connects the back and the abdomen of the human body. In addition, the y-axis direction is a direction from a right hand side to a left hand side of the human body. In addition, the z-axis direction is a direction that connects the head and legs of the human body. In values of the coordinates, an origin is set as the right waist of the human body, and a unit is set as mm. In the example, coordinates of the entrance to the stomach are "(100, 180, 250)." That is, the entrance to the stomach is at a position 100 mm in the x-axis direction, 180 mm in the y-axis direction and 250 mm in the z-axis direction from the right waist. In addition, coordinates of the entrance to the small intestine are "(100, 100, 200)." That is, the entrance to the small intestine is at a position 100 mm in the x-axis direction, 100 mm in the y-axis direction and 200 mm in the z-axis direction from the right waist. In addition, coordinates of the entrance to the large intestine are "(100, 220, 50)." That is, the entrance to the large intestine is at a position 100 mm in the x-axis direction, 220 mm in the y-axis direction and 50 mm in the z-axis direction from the right waist. The x-axis direction, the y-axis direction, the z-axis direction, the origin or the unit of the coordinates are not limited thereto, but may use any value as long as a position of the capsule endoscope 10 may be specified. In addition, before the patient actually swallows the capsule endoscope 10, the coordinate system is set in advance.

Hereinafter, description will return to Fig. 4. In the example shown in Fig. 4, a value stored in the data item "coordinates" in row 101 is "(100, 180, 250)," and a value stored in the data item "frame rate" is "4 frames/s." This means that, when a distance between the capsule endoscope 10 and the position shown by the "coordinates (100, 180, 250)" is less than a predetermined distance threshold, the frame rate of the imaging unit 101 of the capsule endoscope 10 is set to 4 frames per second (4 frame/s). That is, when the distance between the position of the capsule endoscope 10 and the entrance to the stomach is less than the distance threshold, the frame rate of the imaging unit 101 of the capsule endoscope 10 is set to 4 frames per second (4 frame/s).

In addition, in the example shown, a value stored in the data item "coordinates" in row 102 is "(100, 100, 200)," and a value stored in the data item "frame rate" is "2 frames/s." This means that, when a distance between the capsule endoscope 10 and the position shown by the "coordinates (100, 100, 200)" is less than the predetermined distance threshold, the frame rate of the imaging unit 101 of the capsule endoscope 10 is set to 2 frames per second (2 frames/s). That is, when the distance between the position of the capsule endoscope 10 and the entrance to the small intestine is less than the predetermined distance threshold, the frame rate of the imaging unit 101 of the capsule endoscope 10 is set to 2 frames per second (2 frames/s).

In addition, in the example shown, a value stored in the data item "coordinates" in row 103 is "(100, 220, 50)," and a value stored in the data item "frame rate" is "10 frames/s." This means that, when a distance between the capsule endoscope 10 and the position shown by the "coordinates (100, 220, 50)" is less than the predetermined distance threshold, the frame rate of the imaging unit 101 of the capsule endoscope 10 is set to 10 frames per second (10 frames/s). That is, when the distance between the position of the capsule endoscope 10 and the entrance to the large intestine is less than the predetermined distance threshold, the frame rate of the imaging unit 101 of the capsule endoscope 10 is set to 10 frames per second (10 frames/s). The above-mentioned predetermined distance threshold may be previously determined or may be arbitrarily set.

In the example shown in Fig. 4, although three sets of values of the data item "coordinates" showing reference positions of the capsule endoscope 10 and the values of the data item "frame rate" showing frame rates corresponding to the positions of the capsule endoscope 10 are stored, the example is not limited thereto as long as one set or more are stored.

In addition, when imaging of the imaging unit 101 of the capsule endoscope 10 is stopped, the value of the data item "frame rate" is set to "0 frame/s" (0 frame per second = image transmission stop). In addition, before the patient actually swallows the capsule endoscope 10, contents of the position database are set.

Next, a method of estimating the position of the capsule endoscope 10 using the position estimation unit 213 will be described. As described above, when the antenna section 22 is attached to the human body, a location at which the plurality of antenna elements 221 are attached is predetermined. Accordingly, the position estimation unit 213 may estimate a position of the capsule endoscope 10 using a signal level (the received signal intensity) of a signal received by the antenna elements 221 measured by the wireless communication unit 211. In the embodiment, the position estimation unit 213 outputs the position of the capsule endoscope 10 using the coordinates shown in Fig. 5. A method of estimating the position of the capsule endoscope 10 may use any kind of method.

Next, a method of determining the frame rate of the imaging unit 101 of the capsule endoscope 10 using the frame rate-setting unit 215 will be described. The frame rate-setting unit 215 determines the frame rate of the imaging unit 101 of the capsule endoscope 10 as a frame rate stored in association with the coordinates when the distance between the position of the capsule endoscope 10 estimated by the position estimation unit 213 and the position shown by the coordinates stored in the position database is less than the predetermined distance threshold.

Next, an operation of the capsule endoscope 10 will be described. The capsule endoscope 10 periodically transmits a signal (the modulated signal or the non-modulated carrier wave) to the extracorporeal terminal 20. In addition, the capsule endoscope 10 captures an image at the frame rate designated by information that shows the frame rate transmitted from the extracorporeal terminal 20, and transmits the image data to the extracorporeal terminal 20. Accordingly, the capsule endoscope 10 transmits the image data to the extracorporeal terminal 20 at the frame rate set by the extracorporeal terminal 20. The capsule endoscope 10 captures images at a predetermined frame rate and transmits the images to the extracorporeal terminal 20 when no information showing the frame rate is received. The predetermined frame rate may have any value.

Next, an operation of the extracorporeal terminal 20 will be described. The extracorporeal terminal 20 stores the image data transmitted from the capsule endoscope 10 in the image storage unit 212. In addition, the extracorporeal terminal 20 determines the frame rate of the imaging unit 101 of the capsule endoscope 10 and transmits the information showing the determined frame rate to the capsule endoscope 10.

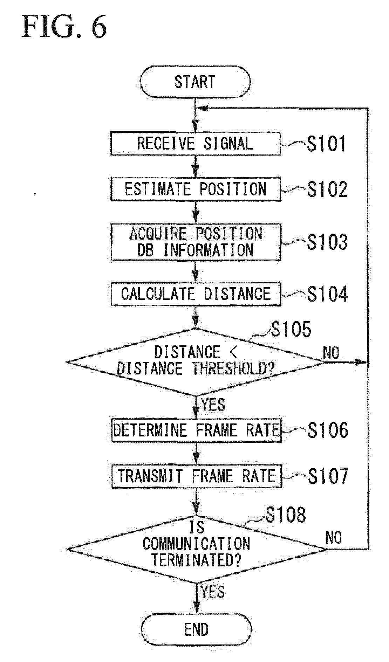

Fig. 6 is a flow chart showing an operational sequence in which the extracorporeal terminal 20 according to the embodiment determines the frame rate of the capsule endoscope 10 and transmits information showing the determined frame rate to the capsule endoscope 10.
(Step S101) The plurality of antenna elements 221 receive signals (the modulated signals or the non-modulated carrier waves) transmitted from the capsule endoscope 10. Next, the wireless communication unit 211 measures signal levels (the received signal intensities) of the signals received by the plurality of antenna elements 221. The operation then proceeds to step S102. The processing of step S101 is referred to as a signal-receiving step.
(Step S102) The position estimation unit 213 estimates a position of the capsule endoscope 10 using the signal levels (received signal intensities) of the signals received by the plurality of antenna elements 221 and measured by the wireless communication unit 211 in the processing of step S101, and outputs the coordinate information to the frame rate-setting unit 215. The processing of step S102 is referred to as a position estimation step.
(Step S103) The frame rate-setting unit 215 reads sets of values stored as the data item "coordinates" and the data item "frame rate" from the position database stored in the storage unit 214. The operation then proceeds to step S104. The processing of step S103 is referred to as a position database information acquisition step.
(Step S104) The frame rate-setting unit 215 calculates distances between two points of the position shown by the coordinate information of the capsule endoscope 10 output from the position estimation unit 213 in the position estimation step of step S102 and the position shown by the value of the data item "coordinates" read in the position database information acquisition step of step S103 for each value of the data item "coordinates." The operation then proceeds to step S105. The processing of step S104 is referred to as a distance calculation step.
(Step S105) The frame rate-setting unit 215 determines whether any one among the distances between the two points calculated in the distance calculation step of step S104 is less than a predetermined distance threshold. When the frame rate-setting unit 215 determines that a distance among the distances between the two points calculated in the distance calculation step of step S104 is less than the predetermined distance threshold, the operation proceeds to step S106, and in the other cases, the operation advances to the processing of step S101. The processing of step S105 is referred to as a threshold comparison and decision step.
(Step S106) The frame rate-setting unit 215 determines the value of the data item "frame rate" stored as a set with the value of the data item "coordinates" as the frame rate of the imaging unit 101 of the capsule endoscope 10 when the distance is less than the predetermined distance threshold among the distances between the two points calculated in the distance calculation step of step S104. The operation then proceeds to step S107. The processing of step S106 is referred to as a frame rate determination step.
(Step S107) The wireless communication unit 211 transmits the frame rate of the imaging unit 101 of the capsule endoscope 10 determined by the frame rate-setting unit 215 in the frame rate determination step of step S 106 to the capsule endoscope 10 via the antenna elements 221 of the antenna section 22. The operation then proceeds to step S108. The processing of step S107 is referred to as a frame rate transmission step.
(Step S108) The wireless communication unit 211 determines whether a signal is received from the capsule endoscope 10 for a specified time or more. When the wireless communication unit 211 determines that the signal is not received from the capsule endoscope 10 for the specified time or more, the processing is terminated, and in the other cases, the operation returns to the processing of step S101. The processing of step S108 is referred to as a termination decision step. For example, when battery exhaustion occurs in the capsule endoscope 10, the signal cannot be received from the capsule endoscope 10. Actually, when it is decided that the signal is not received from the capsule endoscope 10 for the specified time or more, the processing is often terminated.

As described above, according to the embodiment, the position estimation unit 213 of the extracorporeal terminal 20 performs position detection of the capsule endoscope 10 using the signal levels received by the plurality of antenna elements 221 included in the antenna section 22. In addition, the position database stored in the storage unit 214 prestores a set of values of the data item "coordinates" showing reference positions of the capsule endoscope 10 and values of the data item "frame rate" showing frame rates corresponding to the position of the capsule endoscope 10.

According to the embodiment, the frame rate-setting unit 215 determines the values of the data item "frame rate" stored as a set with the values of the data item "coordinates" as the frame rate of the imaging unit 101 of the capsule endoscope 10 when the distance between the position of the capsule endoscope 10 estimated by the position estimation unit 213 and the position showing the values of the data item "coordinates" stored in the position database is less than the predetermined distance threshold. The wireless communication unit 211 then transmits the information showing the determined frame rate of the imaging unit 101 of the capsule endoscope 10 to the capsule endoscope 10 via the antenna elements 221 of the antenna section 22.

According to the embodiment, the imaging unit 101 of the capsule endoscope 10 performs imaging at the frame rate designated by the information showing the frame rate transmitted from the extracorporeal terminal 20. Accordingly, the frame rate of the imaging unit 101 can be modified according to the position of the capsule endoscope 10. For this reason, excessive consumption of the battery of the capsule endoscope 10 can be reduced.

### (Second embodiment)

Next, a second embodiment of the present invention will be described with reference to the accompanying drawings. A capsule endoscope system of the embodiment includes a capsule endoscope and an extracorporeal terminal, as in the first embodiment. In addition, the capsule endoscope according to the embodiment is similar to the capsule endoscope according to the first embodiment. The embodiment differs from the first embodiment in that an extracorporeal terminal of the embodiment includes a distance threshold modification unit.

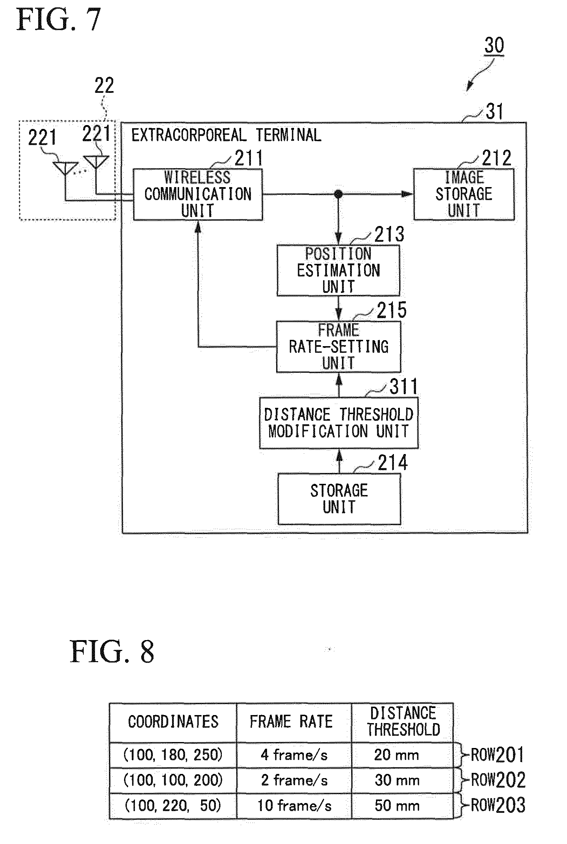

Fig. 7 is a block diagram showing a configuration of an extracorporeal terminal 30 according to the embodiment. In the example shown, the extracorporeal terminal 30 includes a main body section 31 and the antenna section 22. The configuration of the antenna section 22 is similar to that of the antenna section 22 according to the first embodiment. The main body section 31 includes the wireless communication unit 211, the image storage unit 212, the position estimation unit 213, the storage unit 214, the frame rate-setting unit 215 and a distance threshold modification unit 311. The wireless communication unit 211, the image storage unit 212, the position estimation unit 213 and the storage unit 214 are similar to the respective parts of the first embodiment. A configuration of the position database stored in the storage unit 214 is different from that of the first embodiment. The configuration of the position database of the embodiment will be described below.

The distance threshold modification unit 311 modifies distance thresholds with respect to the data sets of the coordinates and the frame rates acquired from the storage unit 214 and outputs the modified distance thresholds to the frame rate-setting unit 215. The frame rate-setting unit 215 calculates a distance between a frame rate modification position acquired from the distance threshold modification unit 311 and an estimated position of the capsule endoscope 10 acquired from the position estimation unit 213, and determines modification to the corresponding frame rate when the calculated distance is less than the distance threshold acquired from the distance threshold modification unit 311.

Next, the position database will be described. Fig. 8 is a schematic view showing a data structure of the position database according to the embodiment. The position database has data items such as "coordinates," "frame rate" and "distance threshold," and stores data of the data items in every row in association with each other. The data item "coordinates" stores coordinates showing a position at which a frame rate of the imaging unit 101 of the capsule endoscope 10 is set. The data item "frame rate" stores the frame rate of the imaging unit 101 of the capsule endoscope 10. The data item "distance threshold" stores a distance threshold.

A relationship between the coordinates of the embodiment and the human body is similar to the relationship between the coordinates of the first embodiment and the human body. In the example shown, a value stored in a data item "coordinates" in row 201 is "(100, 180, 250)," a value stored in a data item "frame rate" is "4 frames/s" and a distance threshold is "20 mm." This means that, when a distance between the capsule endoscope 10 and the "coordinates (100, 180, 250)" is less than "20 mm," the frame rate of the imaging unit 101 of the capsule endoscope 10 is set to 4 frames per second (4 frames/s). That is, when the distance between the position of the capsule endoscope 10 and the entrance to the stomach is less than 20 mm, the frame rate of the imaging unit 101 of the capsule endoscope 10 is set to 4 frames per second (4 frames/s).

In addition, in the example shown, a value stored in the data item "coordinates" in row 202 is "(100, 100, 200)," a value stored in the data item "frame rate" is "2 frames/s" and a value stored in the data item "distance threshold" is "30 mm." This means that, when the distance between the capsule endoscope 10 and the "coordinates (100, 100, 200)" is less than 30 mm, the frame rate of the imaging unit 101 of the capsule endoscope 10 is set to 2 frames per second (2 frames/s). That is, when the distance between the position of the capsule endoscope 10 and the entrance to the small intestine is less than 30 mm, the frame rate of the imaging unit 101 of the capsule endoscope 10 is set to 2 frames per second (2 frames/s).

In addition, in the example shown, a value stored in the data item "coordinates" in row 203 is "(100, 220, 50)," a value stored in the data item "frame rate" is "10 frames/s" and a value stored in the data item "distance threshold" is "50 mm." This means that, when the distance between the capsule endoscope 10 and the "coordinates (100, 220, 50)" is less than 50 mm, the frame rate of the imaging unit 101 of the capsule endoscope 10 is set to 10 frames per second (10 frames/s). That is, when the distance between the position of the capsule endoscope 10 and the entrance to the large intestine is less than 50 mm, the frame rate of the imaging unit 101 of the capsule endoscope 10 is set to 10 frames per second (10 frames/s).

In the example shown in Fig. 8, three sets of values of the data item "coordinates" showing the reference positions of the capsule endoscope 10, the values of the data item "frame rate" showing the frame rates corresponding to the positions of the capsule endoscope 10 and the values of the data item "distance threshold" showing the distance thresholds are shown, the embodiment is not limited thereto as long as one set or more is stored. In addition, although the values of the data item "distance threshold" are different among the three sets, the values may be equal to each other. In addition, when the imaging of the imaging unit 101 of the capsule endoscope 10 is stopped, the value of the data item "frame rate" is set to "0 frame/s" (0 frame per second = image transmission stop). In addition, before the patient actually swallows the capsule endoscope 10, contents of the position database are set.

Next, a method of determining the frame rate of the imaging unit 101 of the capsule endoscope 10 using the frame rate-setting unit 215 will be described. The frame rate-setting unit 215 determines the frame rate of the imaging unit 101 of the capsule endoscope 10 as a frame rate stored in association with the coordinates when the distance between the position of the capsule endoscope 10 estimated by the position estimation unit 213 and the position shown by the coordinates stored in the position database is less than the distance threshold stored in the position database. The distance threshold is modified by the distance threshold modification unit 311.

Next, an operation of the capsule endoscope 10 will be described. The operation of the capsule endoscope 10 is similar to that of the capsule endoscope 10 of the first embodiment.

Next, an operation of the extracorporeal terminal 30 will be described. The extracorporeal terminal 30 stores the image data transmitted from the capsule endoscope 10 in the image storage unit 212. In addition, the extracorporeal terminal 30 determines the frame rate of the imaging unit 101 of the capsule endoscope 10, and transmits the information showing the determined frame rate to the capsule endoscope 10.

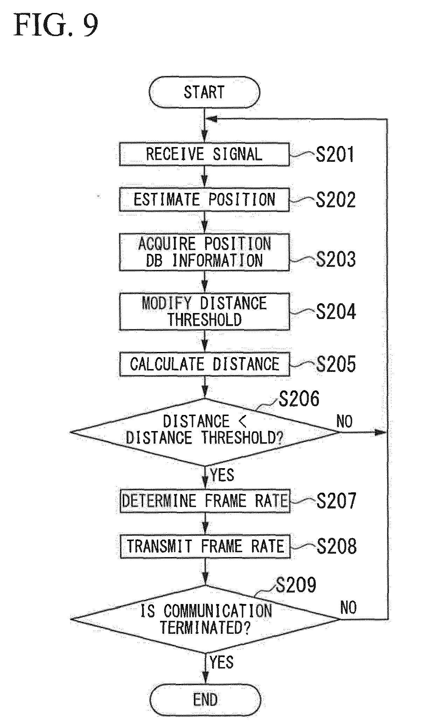

Fig. 9 is a flow chart showing an operational sequence in which the extracorporeal terminal 30 of the embodiment determines the frame rate of the capsule endoscope 10 and transmits the information showing the determined frame rate to the capsule endoscope 10.

Processing of step S201 to step S202 is similar to the processing of step S101 to step S102 of the first embodiment.
(Step S203) The distance threshold modification unit 311 reads a set of values stored in the data item "coordinates," the data item "frame rate" and the data item "distance threshold" from the position database stored in the storage unit 214. In addition, the distance threshold modification unit 311 outputs the set of values of the read data item "coordinates" and the read data item "frame rate" to the frame rate-setting unit 215. The operation then proceeds to step S204. Processing of step S203 is referred to as a position database information acquisition step.
(Step S204) The distance threshold modification unit 311 outputs the value of the data item "distance threshold" read in the processing of step S203 to the frame rate-setting unit 215. The operation then proceeds to step S205. The processing of step S204 is referred to as a distance threshold modification step.
(Step S205) The frame rate-setting unit 215 calculates distances between two points of the position shown by the coordinate information of the capsule endoscope 10 output by the position estimation unit 213 in the position estimation step of step S202 and the position shown by the value of the data item "coordinates" acquired in the position database information acquisition step of step S203 for each value of the data item "coordinates." The operation then proceeds to step S206. The processing of step S205 is referred to as a distance calculation step.
(Step S206) The frame rate-setting unit 215 determines whether any one of the distances between the two points calculated in the distance calculation step of step S205 is less than the value of the data item "distance threshold" acquired in the processing of step S204. When the frame rate-setting unit 215 determines that a distance among the distances between the two points calculated in the distance calculation step of step S205 is less than the value of the data item "distance threshold" acquired in the processing of step S204, the operation proceeds to step S207, and in the other cases, the operation returns to the processing of step S201. The processing of step S206 is referred to as a threshold comparison and decision step.

Processing of step S207 to step S209 is similar to the processing of step S 106 to step S108 of the first embodiment.

As described above, according to the embodiment, the position estimation unit 213 of the extracorporeal terminal 30 performs position detection of the capsule endoscope 10 using the signal levels received by the plurality of antenna elements 221 included in the antenna section 22. In addition, the position database stored in the storage unit 214 prestores sets of values of the data item "coordinates" showing reference positions of the capsule endoscope 10, values of the data item "frame rate" showing frame rates corresponding to the position of the capsule endoscope 10 and values of the data item "distance threshold" showing distance thresholds.

According to the embodiment, the frame rate-setting unit 215 determines the values of the data item "frame rate" stored as a set with the values of the data item "coordinates" as the frame rate of the imaging unit 101 of the capsule endoscope 10 when the distance between the position of the capsule endoscope 10 estimated by the position estimation unit 213 and the position showing the value of the data item "coordinates" stored in the position database is less than the value of the data item "distance threshold" stored as a set of the values with the data item "coordinates." The wireless communication unit 211 then transmits the information showing the determined frame rate of the imaging unit 101 of the capsule endoscope 10 to the capsule endoscope 10 via the antenna elements 221 of the antenna section 22.

According to the embodiment, the imaging unit 101 of the capsule endoscope 10 performs imaging at the frame rate designated by the information showing the frame rate transmitted from the extracorporeal terminal 30. Accordingly, since the frame rate of the imaging unit 101 according to the position of the capsule endoscope 10 can be modified, consumption of an extra battery of the capsule endoscope 10 can be reduced. In addition, by changing the distance threshold in response to positional variation due to movement of internal organs in the body cavity or lumen thicknesses of the internal organs, even when deviation occurs in the positional variation or the lumen thickness, a more appropriate frame rate modification can be provided to the capsule endoscope 10.

### (Third embodiment)

Next, a third embodiment of the present invention will be described with reference to the accompanying drawings. A capsule endoscope system according to the embodiment includes a capsule endoscope and an extracorporeal terminal, as in the first embodiment. In addition, the capsule endoscope according to the embodiment is similar to the capsule endoscope according to the first embodiment. The embodiment differs from the first embodiment in that an extracorporeal terminal of the embodiment includes a distance threshold modification unit and a speed calculation unit.

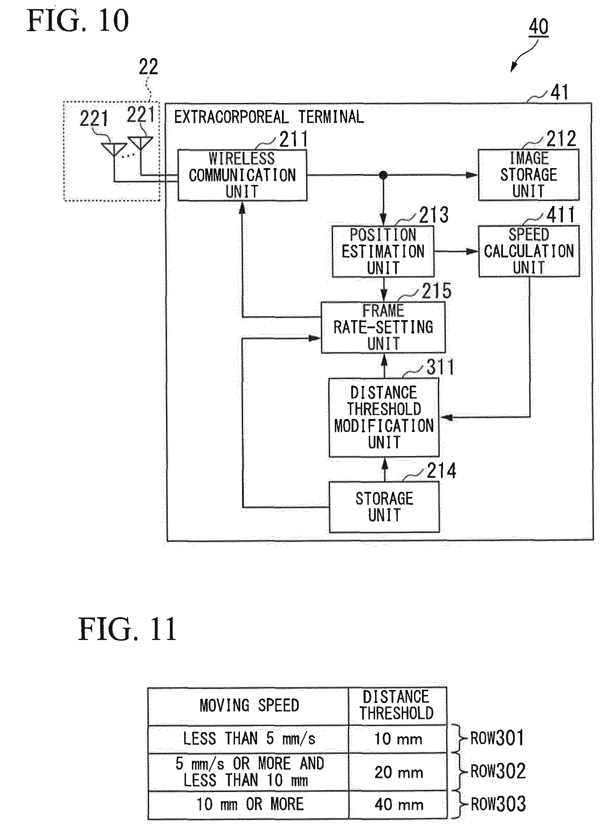

Fig. 10 is a block diagram showing a configuration of an extracorporeal terminal 40 according to the embodiment. In the example shown, the extracorporeal terminal 40 includes a main body section 41 and the antenna section 22. A configuration of the antenna section 22 is similar to that of the antenna section 22 of the first embodiment. The main body section 41 includes the wireless communication unit 211, the image storage unit 212, the position estimation unit 213, the storage unit 214, the frame rate-setting unit 215, the distance threshold modification unit 311 and a speed calculation unit 411. The wireless communication unit 211, the image storage unit 212, the position estimation unit 213 and the storage unit 214 are similar to the respective parts of the first embodiment. The storage unit 214 stores a distance threshold table in addition to the position database. A configuration of the position database is similar to that of the position database of the first embodiment. A configuration of the distance threshold table will be described below.

The speed calculation unit 411 calculates a moving speed of the capsule endoscope 10 based on a distance of the capsule endoscope 10 between two points estimated by the position estimation unit 213 and a time of movement between the two points.

The distance threshold modification unit 311 sets a distance threshold based on the moving speed of the capsule endoscope 10 calculated by the speed calculation unit 411 and the distance threshold table stored in the storage unit 214. A method of setting the distance threshold will be described below. The frame rate-setting unit 215 determines a frame rate of the imaging unit 101 of the capsule endoscope 10 based on the position database stored in the storage unit 214, the position of the capsule endoscope 10 estimated by the position estimation unit 213 and the distance threshold set by the distance threshold modification unit 311. A method of determining the frame rate will be described below.

Next, the distance threshold table will be described. Fig. 11 is a schematic view showing a data structure of the distance threshold table of the embodiment. The distance threshold table has data items of "moving speed" and "distance threshold" and stores data of the data items in association with each row.

The data item "moving speed" stores a speed of the capsule endoscope 10. The data item "distance threshold" stores a distance threshold.

In the example shown, a value stored in the data item "moving speed" in row 301 is "less than 5 mm/s" and the value stored in the data item "distance threshold" is "10 mm." Thus, when the moving speed of the capsule endoscope 10 is less than 5 mm per second, the distance threshold may be set to 10 mm.

In addition, in the example shown, a value stored in the data item "moving speed" in row 302 is "5 mm/s to less than 10 mm/s" and the value stored in the data item "distance threshold" is "20 mm." Thus, when the moving speed of the capsule endoscope 10 is 5 mm or more and less than 10 mm per second, the distance threshold is set to 20 mm.

In addition, in the example shown, a value stored in the data item "moving speed" in row 303 is "10 mm/s or more" and the value stored in the data item "distance threshold" is "40 mm." Thus, when the moving speed of the capsule endoscope 10 is 10 mm or more per second, the distance threshold is set to 40 mm.

In the example shown in Fig. 11, although three sets of values of the data item "moving speed" and the values of the data item "distance threshold" are stored, the example is not limited thereto. In addition, before the patient actually swallows the capsule endoscope 10, contents of the distance threshold table are set.

Next, a method of setting a distance threshold using the distance threshold modification unit 311 will be described. The distance threshold modification unit 311 acquires the moving speed of the capsule endoscope 10 calculated by the speed calculation unit 411 and sets the value associated with the acquired speed among the distance thresholds stored in the distance threshold table as a distance threshold. For example, the distance threshold modification unit 311 sets the distance threshold to 10 mm when the moving speed of the capsule endoscope 10 calculated by the speed calculation unit 411 is 3mm per second.

Next, a method of determining the frame rate of the imaging unit 101 of the capsule endoscope 10 using the frame rate-setting unit 215 will be described. The frame rate-setting unit 215 determines the frame rate of the imaging unit 101 of the capsule endoscope 10 as the frame rate stored in association with the coordinates when the distance between the position of the capsule endoscope 10 estimated by the position estimation unit 213 and the position showing the coordinates stored in the position database is less than the distance threshold set by the distance threshold modification unit 311.

Next, an operation of the capsule endoscope 10 will be described. The operation of the capsule endoscope 10 is similar to that of the capsule endoscope 10 of the embodiment.

Next, an operation of the extracorporeal terminal 40 will be described. The extracorporeal terminal 40 stores the image data transmitted from the capsule endoscope 10 in the image storage unit 212. In addition, the extracorporeal terminal 40 determines the frame rate of the imaging unit 101 of the capsule endoscope 10 and transmits the information showing the determined frame rate to the capsule endoscope 10.

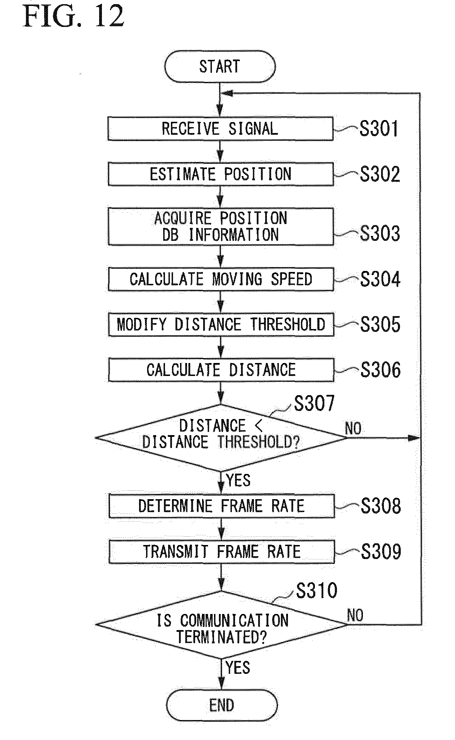

Fig. 12 is a flow chart showing an operational sequence in which the extracorporeal terminal 40 of the embodiment determines the frame rate of the capsule endoscope 10 and transmits the information showing the determined frame rate to the capsule endoscope 10.

Processing of step S301 to step S303 is similar to the processing of step S101 to step S103 of the first embodiment.
(Step S304) The speed calculation unit 411 calculates a moving speed of the capsule endoscope 10 based on a chronological change of the coordinates of the estimated position of the capsule endoscope 10 estimated by the position estimation unit 213. For example, the speed calculation unit 411 calculates a moving speed of the capsule endoscope 10 based on the coordinates of the capsule endoscope 10 estimated by the position estimation unit 213 in the processing of step S302, the coordinates of the capsule endoscope 10 estimated by the position estimation unit 213 in the processing of step S302 performed one time before the processing of step S302 in which the coordinates are estimated, and a time required for movement between the two coordinates. The operation then proceeds to step S305. The processing of step S304 is referred to as a speed calculation step.
(Step S305) The distance threshold modification unit 311 sets a distance threshold based on the moving speed of the capsule endoscope 10 calculated by the speed calculation unit 411 in the speed calculation step of step S304 and the distance threshold table stored in the storage unit 214. The operation then proceeds to step S306. The processing of step S305 is referred to as a distance threshold modification step.
(Step S306) The frame rate-setting unit 215 calculates distances between the two points of the position shown by the coordinate information of the capsule endoscope 10 output from the position estimation unit 213 in the position estimation step of step S302 and the position shown by the value of the data item "coordinates" read in the position database information acquisition step of step S303 for each value of the data item "coordinates." The operation then proceeds to step S307. The processing of step S306 is referred to as a distance calculation step.
(Step S307) The frame rate-setting unit 215 determines whether any one of the distances between the two points calculated in the distance calculation step of step S306 is less than the distance threshold set by the distance threshold modification unit 311 in the distance threshold modification step of step S305. When the frame rate-setting unit 215 determines that a distance among the distances between the two points calculated in the distance calculation step of step S306 is less than the distance threshold set by the distance threshold modification unit 311 in the distance threshold modification step of step S305, the operation proceeds to step S308, and in the other cases, the operation returns to the processing of step S301. The processing of step S307 is referred to as a threshold comparison and decision step.

The processing of step S308 to step S310 is similar to the processing of step S 106 to step S108 of the first embodiment.

As described above, according to the embodiment, the position estimation unit 213 of the extracorporeal terminal 40 performs position detection of the capsule endoscope 10 using the signal levels received by the plurality of antenna elements 221 included in the antenna section 22. In addition, the position database stored in the storage unit 214 prestores a set of values of the data item "coordinates" showing reference positions of the capsule endoscope 10 and values of the data item "frame rate" showing frame rates corresponding to the position of the capsule endoscope 10. In addition, the distance threshold table stored in the storage unit 214 prestores sets of values of the data item "moving speed" showing moving speeds of the capsule endoscope 10 and values of the data item "distance threshold" showing distance thresholds.

According to the embodiment, the speed calculation unit 411 calculates a moving speed of the capsule endoscope 10 based on the chronological change of the coordinates of the estimated position of the capsule endoscope 10 estimated by the position estimation unit 213. Then, the distance threshold modification unit 311 sets a distance threshold based on the moving speed of the capsule endoscope 10 calculated by the speed calculation unit 411 and the distance threshold table stored in the storage unit 214.

According to the embodiment, the frame rate-setting unit 215 determines the value of the data item "frame rate" stored as the set with the value of the data item "coordinates" as the frame rate of the imaging unit 101 of the capsule endoscope 10 when the distance between the position of the capsule endoscope 10 estimated by the position estimation unit 213 and the position shown by the value of the data item "coordinates" stored in the position database is less than the distance threshold set by the distance threshold modification unit 311. The wireless communication unit 211 then transmits the information showing the determined frame rate of the imaging unit 101 of the capsule endoscope 10 to the capsule endoscope 10 via the antenna elements 221 of the antenna section 22.

According to the embodiment, the imaging unit 101 of the capsule endoscope 10 performs imaging at the frame rate designated by the information showing the frame rate transmitted from the extracorporeal terminal 40. Accordingly, since the frame rate of the imaging unit 101 is modified according to the position of the capsule endoscope 10, excessive consumption of the battery of the capsule endoscope 10 can be reduced. In addition, as the distance threshold is modified according to the moving speed of the capsule endoscope 10 in the body cavity, even when the moving speed of the capsule endoscope 10 is high, the modification of the frame rate can be reliably performed.

### (Fourth embodiment)

Next, a fourth embodiment of the present invention will be described with reference to the accompanying drawings. A capsule endoscope system of the embodiment includes a capsule endoscope and an extracorporeal terminal, as in the first embodiment. In addition, the capsule endoscope according to the embodiment is similar to the capsule endoscope according to the first embodiment. The embodiment differs from the first embodiment in that an extracorporeal terminal according to the embodiment includes a distance threshold modification unit and an image variation calculation unit.

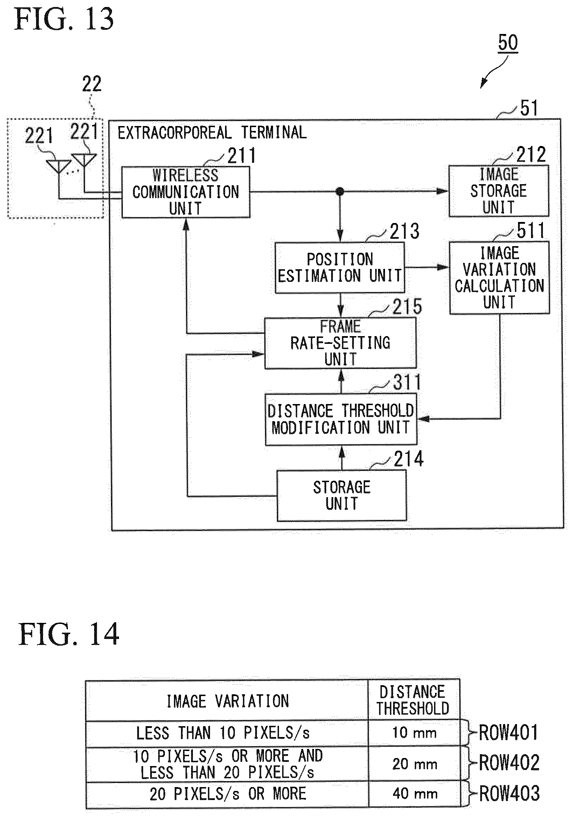

Fig. 13 is a block diagram showing a configuration of an extracorporeal terminal 50 of the embodiment. In an example shown, the extracorporeal terminal 50 includes a main body section 51 and the antenna section 22. A configuration of the antenna section 22 is similar to that of the antenna section 22 of the first embodiment. The main body section 51 includes the wireless communication unit 211, the image storage unit 212, the position estimation unit 213, the storage unit 214, the frame rate-setting unit 215, the distance threshold modification unit 311 and an image variation calculation unit 511. The wireless communication unit 211, the image storage unit 212, the position estimation unit 213 and the storage unit 214 are similar to the respective parts of the first embodiment. The storage unit 214 stores a distance threshold table in addition to the position database. A configuration of the position database is similar to that of the position database of the first embodiment. A configuration of the distance threshold table will be described below.

The image variation calculation unit 511 calculates an image variation using the image data stored in the image storage unit 212. Here, the image variation is a chronological variation between the latest image data and the image data one or more frame before it. For example, the image variation may be a moving quantity of an attention region extracted by performing pattern matching between an acquired image data and an affected area information database (not shown) that is preregistered. The distance threshold modification unit 311 sets a distance threshold based on the image variation calculated by the image variation calculation unit 511 and the distance threshold table stored in the storage unit 214. A method of setting the distance threshold will be described below. The frame rate-setting unit 215 determines the frame rate of the imaging unit 101 of the capsule endoscope 10 based on the position database stored in the storage unit 214, the position of the capsule endoscope 10 estimated by the position estimation unit 213 and the distance threshold set by the distance threshold modification unit 311. A method of determining the frame rate will be described below.

Next, the distance threshold table will be described. Fig. 14 is a schematic view showing a data structure of the distance threshold table according to the embodiment. The distance threshold table has data items of "image variation" and "distance threshold," and stores data of the data items in association with each row. The data item "image variation" stores an image variation. The data item "distance threshold" stores a distance threshold.

In the example shown, a value stored in the data item "image variation" in row 401 is "less than 10 pixels/s" and a value stored in the data item "distance threshold" is "10 mm." Thus, when the image variation is less than 10 pixels per second, the distance threshold is set to 10 mm.

In addition, in the example shown, a value stored in the data item "image variation" in row 402 is "10 pixels/s or more and less than 20 pixels/s" and a value stored in the data item "distance threshold" is "20 mm." Thus, when the image variation is 10 pixels or more and less than 20 pixels per second, the distance threshold is set to 20 mm.

In addition, in the example shown, a value stored in the data item "image variation" in row 403 is "20 pixels/s or more" and a value stored in the data item "distance threshold" is "40 mm." Thus, when the image variation is 20 pixels or more per second, the distance threshold is set to 40 mm.

In the example shown in Fig. 11, although three sets of values of the data item "image variation" and the values of the data item "distance threshold" are stored, the example is not limited thereto. In addition, before the patient actually swallows the capsule endoscope 10, contents of the distance threshold table are set.

Next, a method of setting the distance threshold using the distance threshold modification unit 311 will be described. The distance threshold modification unit 311 acquires the image variation calculated by the image variation calculation unit 511 and sets the value associated with the acquired image variation among the distance thresholds stored in the distance threshold table as a distance threshold. For example, the distance threshold modification unit 311 sets the distance threshold to 10 mm when the image variation calculated by the image variation calculation unit 511 is 3 pixels per second.

Next, a method of determining the frame rate of the imaging unit 101 of the capsule endoscope 10 using the frame rate-setting unit 215 will be described. The frame rate-setting unit 215 determines the frame rate of the imaging unit 101 of the capsule endoscope 10 as the frame rate stored in association with the coordinates when the distance between the position of the capsule endoscope 10 estimated by the position estimation unit 213 and the position shown by the coordinates stored in the position database is less than the distance threshold set by the distance threshold modification unit 311.

Next, an operation of the capsule endoscope 10 will be described. The operation of the capsule endoscope 10 is similar to that of the capsule endoscope 10 according to the first embodiment.

Next, an operation of the extracorporeal terminal 50 will be described. The extracorporeal terminal 50 stores the image data transmitted from the capsule endoscope 10 in the image storage unit 212. In addition, the extracorporeal terminal 50 determines the frame rate of the imaging unit 101 of the capsule endoscope 10 and transmits the information showing the determined frame rate to the capsule endoscope 10.

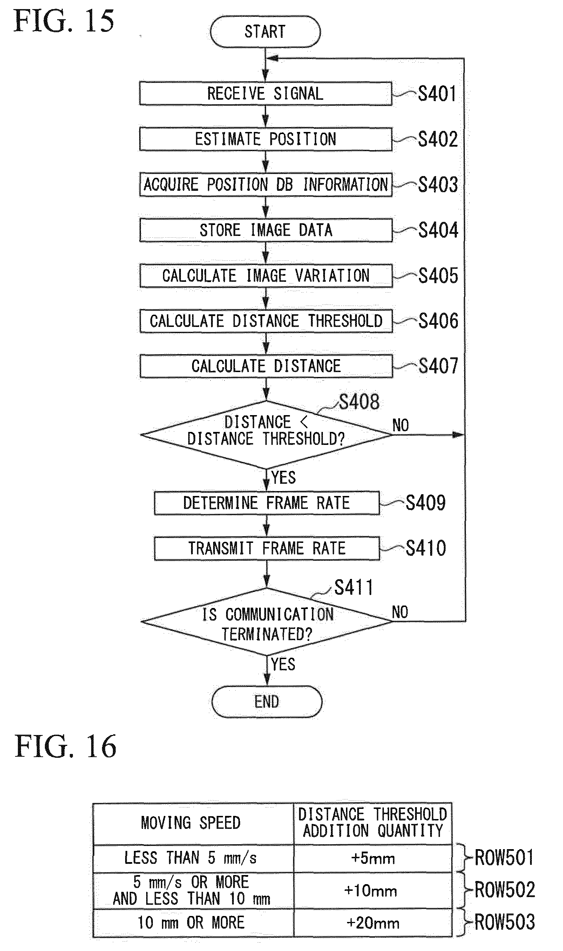

Fig. 15 is a flow chart showing an operational sequence in which the extracorporeal terminal 50 of the embodiment determines the frame rate of the capsule endoscope 10 and transmits the information showing the determined frame rate to the capsule endoscope 10.

Processing of step S401 to step S403 is similar to the processing of step S101 to step S103 according to the first embodiment.
(Step S404) The image storage unit 212 stores the image data received from the capsule endoscope 10 by the wireless communication unit 211. The operation then proceeds to step S405. Processing of step S404 is referred to as an image storage step.
(Step S405) The image variation calculation unit 511 calculates an image variation using the image data stored in the image storage unit 212. The operation then proceeds to step S406. The processing of step S405 is referred to as an image variation calculation step.
(Step S406) The distance threshold modification unit 311 sets a distance threshold based on the image variation calculated by the image variation calculation unit 511 in the image variation calculation step of step S405 and the distance threshold table stored in the storage unit 214. The operation then proceeds to step S407. The processing of step S406 is referred to as a distance threshold modification step.
(Step S407) The frame rate-setting unit 215 calculates distances between the two points of the position shown by the coordinate information of the capsule endoscope 10 output from the position estimation unit 213 in the position estimation step of step S402 and the position shown by the value of the data item "coordinates" read in the position database information acquisition step of step S403 for each value of the data item "coordinates." The operation then proceeds to step S408. The processing of step S407 is referred to as a distance calculation step.
(Step S408) The frame rate-setting unit 215 determines whether any one of the distances between the two points calculated in the distance calculation step of step S407 is less than the distance threshold set by the distance threshold modification unit 311 in the distance threshold modification step of step S406. When the frame rate-setting unit 215 determines that a distance among the distances between the two points calculated in the distance calculation step of step S407 is less than the distance threshold set by the distance threshold modification unit 311 in the distance threshold modification step of step S406, the operation proceeds to step S409, and in the other cases, the operation returns to the processing of step S401. The processing of step S408 is referred to as a threshold comparison and decision step.

Processing of step S409 to step S411 is similar to the processing of step S 106 to step S108 according to the first embodiment.

As described above, according to the embodiment, the position estimation unit 213 of the extracorporeal terminal 50 performs position detection of the capsule endoscope 10 using the signal levels received by the plurality of antenna elements 221 included in the antenna section 22. In addition, the position database stored in the storage unit 214 prestores sets of values of the data item "coordinates" showing reference positions of the capsule endoscope 10 and values of the data item "frame rate" showing frame rates corresponding to the position of the capsule endoscope 10. In addition, the distance threshold table stored in the storage unit 214 prestores sets of values of the data item "image variation" showing image variations and values of the data item "distance threshold" showing distance thresholds.

According to the embodiment, the image variation calculation unit 511 calculates the image variation using the image data stored in the image storage unit 212. Then, the distance threshold modification unit 311 sets a distance threshold based on the image variation calculated by the image variation calculation unit 511 and the distance threshold table stored in the storage unit 214.

According to the embodiment, the frame rate-setting unit 215 determines the value of the data item "frame rate" stored as the set with the value of the data item "coordinates" as the frame rate of the imaging unit 101 of the capsule endoscope 10 when the distance between the position of the capsule endoscope 10 estimated by the position estimation unit 213 and the position shown by the value of the data item "coordinates" stored in the position database is less than the distance threshold set by the distance threshold modification unit 311. The wireless communication unit 211 then transmits the information showing the determined frame rate of the imaging unit 101 of the capsule endoscope 10 to the capsule endoscope 10 via the antenna elements 221 of the antenna section 22.

According to the embodiment, the imaging unit 101 of the capsule endoscope 10 performs imaging at the frame rate designated by the information showing the frame rate transmitted from the extracorporeal terminal 50. Accordingly, since the frame rate of the imaging unit 101 can be modified according to the position of the capsule endoscope 10, excessive consumption of the battery of the capsule endoscope 10 can be reduced. In addition, as the distance threshold is modified according to the image variation of the capsule endoscope 10 in a body cavity, even when the variation of the image data acquired by the capsule endoscope 10 is large, modification of the frame rate can be reliably performed.

### (Fifth embodiment)

Next, a fifth embodiment of the present invention will be described with reference to the accompanying drawings. A capsule endoscope system according to the embodiment includes the capsule endoscope 10 and the extracorporeal terminal 40, as in the third embodiment. In addition, the capsule endoscope 10 according to the embodiment is similar to the capsule endoscope 10 according to the third embodiment. In addition, the extracorporeal terminal 40 according to the embodiment is similar to the extracorporeal terminal 40 according to the third embodiment. The embodiment differs from the third embodiment in that the storage unit 214 of the extracorporeal terminal 40 stores a distance threshold addition table and a position database has a different configuration. The configuration of the position database of the embodiment is similar to that of the position database of the second embodiment.

Next, a distance threshold addition table will be described. Fig. 16 is a schematic view showing a data structure of the distance threshold addition table of the embodiment. The distance threshold addition table has data items of "moving speed" and "distance threshold addition quantity" and stores data of the data items at each row. The data item "moving speed" stores a speed of the capsule endoscope 10. The data item "distance threshold addition quantity" stores a distance threshold addition quantity.

In the example shown, a value stored in the data item "moving speed" in row 501 is "less than 5 mm/s" and a value stored in the data item "distance threshold addition quantity" is "+5 mm." Thus, when the moving speed of the capsule endoscope 10 is less than 5 mm per second, 5 mm is added to the distance threshold.

In addition, in the example shown, a value stored in the data item "moving speed" in row 502 is "5 mm/s or more and less than 10 mm/s" and a value stored in the data item "distance threshold addition quantity" is "+10 mm." Thus, when the moving speed of the capsule endoscope 10 is 5 mm or more and less than 10 mm per second, 10 mm is added to the distance threshold.

In addition, in the example shown, a value stored in the data item "moving speed in row 503 is "10 mm/s or more" and a value stored in the data item "distance threshold addition quantity" is "+20 mm." This means that, when a moving speed of the capsule endoscope 10 is 10 mm or more per second, 20 mm is added to the distance threshold.

In the example shown in Fig. 16, although three sets of values of the data item "moving speed" and the values of the data item "distance threshold addition quantity" are stored, the example is not limited thereto. In addition, before the patient actually swallows the capsule endoscope 10, contents of the distance threshold addition table are set.

Next, a method of setting the distance threshold addition quantity using the distance threshold modification unit 311 will be described. The distance threshold modification unit 311 acquires the moving speed of the capsule endoscope 10 calculated by the speed calculation unit 411 and sets the value associated with the acquired speed among the distance threshold addition quantities stored in the distance threshold addition table as a distance threshold addition quantity. For example, the distance threshold modification unit 311 sets the distance threshold addition quantity to 5 mm when the moving speed of the capsule endoscope 10 calculated by the speed calculation unit 411 is 3 mm per second.

Next, a method of setting the distance threshold using the distance threshold modification unit 311 will be described. The distance threshold modification unit 311 sets a value obtained by adding the set distance threshold addition quantity to the value of the data item "distance threshold" of the position database as a distance threshold. For example, when the distance threshold addition quantity is 5 mm, the distance threshold modification unit 311 sets the distance threshold in the case of using the value (100, 180, 250) of the data item "coordinates" to 20 mm + 5 mm = 25 mm, sets the distance threshold in the case of using the value (100, 100, 200) of the data item "coordinates" to 30 mm + 5 mm = 35 mm, and sets the distance threshold in the case of using the value (100, 220, 50) of the data item "coordinates" to 20 mm + 5 mm = 25.

Next, a method of determining the frame rate of the imaging unit 101 of the capsule endoscope 10 using the frame rate-setting unit 215 will be described. The frame rate-setting unit 215 determines the frame rate of the imaging unit 101 of the capsule endoscope 10 as a frame rate stored in association with the coordinates when the distance between the position of the capsule endoscope 10 estimated by the position estimation unit 213 and the position shown by the coordinates stored in the position database is less than the distance threshold set by the distance threshold modification unit 311.

Next, an operation of the capsule endoscope 10 will be described. The operation of the capsule endoscope 10 is similar to that of the capsule endoscope 10 of the first embodiment.

Next, an operation of the extracorporeal terminal 40 will be described. The extracorporeal terminal 40 stores the image data transmitted from the capsule endoscope 10 in the image storage unit 212. In addition, the extracorporeal terminal 40 determines the frame rate of the imaging unit 101 of the capsule endoscope 10 and transmits the information showing the determined frame rate to the capsule endoscope 10.

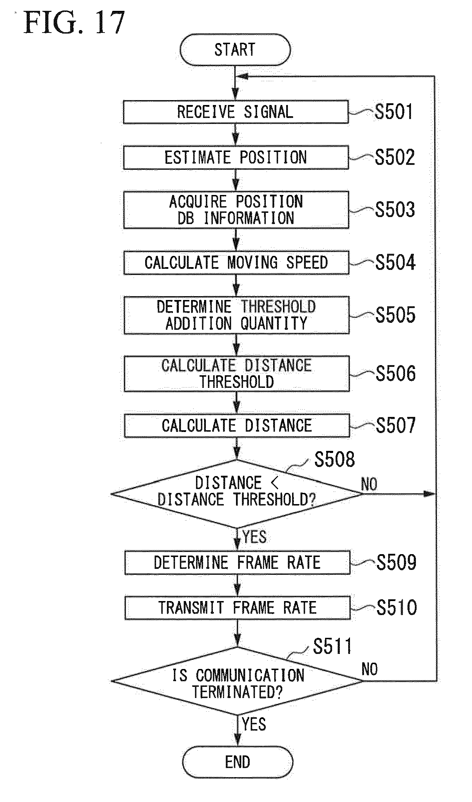

Fig. 17 is a flow chart showing an operational sequence in which the extracorporeal terminal 40 of the embodiment determines the frame rate of the capsule endoscope 10 and transmits the information showing the determined frame rate to the capsule endoscope 10.

Processing of step S501 to step S503 is similar to the processing of step S101 to step S103 of the first embodiment.
(Step S504) The speed calculation unit 411 calculates a moving speed of the capsule endoscope 10 based on a chronological change of the coordinates of the estimated position of the capsule endoscope 10 estimated by the position estimation unit 213. For example, the speed calculation unit 411 calculates a moving speed of the capsule endoscope 10 based on the coordinates of the capsule endoscope 10 estimated by the position estimation unit 213 in the processing of step S502, and the coordinates of the capsule endoscope 10 estimated by the position estimation unit 213 in the processing of step S502 performed once before the processing of step S502 in which the coordinates are estimated, and a time required for movement between the two coordinates. The operation then advance to processing of step S505. Processing of step S504 is referred to as a speed calculation step.
(Step S505) The distance threshold modification unit 311 sets a distance threshold addition quantity based on the moving speed of the capsule endoscope 10 calculated by the speed calculation unit 411 in the speed calculation step of step S504 and the distance threshold addition table stored in the storage unit 214. The operation then proceeds to step S506. The processing of step S505 is referred to as a distance threshold addition quantity determination step.
(Step S506) The distance threshold modification unit 311 sets a value obtained by adding the distance threshold addition quantity set in the distance threshold addition quantity determination step of step S505 to each value of the data item "distance threshold" of the position database as a distance threshold. The operation then proceeds to step S507. The processing of step S506 is referred to as a distance threshold modification step.
(Step S507) The frame rate-setting unit 215 calculates the distance between the two points of the position shown by the coordinate information of the capsule endoscope 10 output from the position estimation unit 213 in the position estimation step of step S502 and the position shown by the value of the data item "coordinates" read in the position database information acquisition step of step S503 for each value of the data item "coordinates." The operation then proceeds to step S508. The processing of step S507 is referred to as a distance calculation step.
(Step S508) The frame rate-setting unit 215 determines whether any one of the distances between the two points calculated in the distance calculation step of step S507 is less than the distance threshold set by the distance threshold modification unit 311 in the distance threshold modification step of step S506. When the frame rate-setting unit 215 determines that a distance among the distances between the two points calculated in the distance calculation step of step S507 is less than the distance threshold set by the distance threshold modification unit 311 in the distance threshold modification step of step S506, the operation proceeds to step S509, and in the other cases, the operation returns to the processing of step S501. The processing of step S508 is referred to as a threshold comparison and decision step.

Processing of step S509 to step S511 is similar to the processing of step S 106 to step S108 of the first embodiment.

As described above, according to the embodiment, the position estimation unit 213 of the extracorporeal terminal 40 performs position detection of the capsule endoscope 10 using the signal levels received by the plurality of antenna elements 221 included in the antenna section 22. In addition, the position database stored in the storage unit 214 prestores sets of values of the data item "coordinates" showing reference positions of the capsule endoscope 10, values of the data item "frame rate" showing frame rates corresponding to the position of the capsule endoscope 10, and values of the data item "distance threshold" showing distance thresholds. In addition, the distance threshold addition table stored in the storage unit 214 prestores sets of values of the data item "moving speed" showing moving speeds of the capsule endoscope 10 and values of the data item "distance threshold addition quantity" showing distance threshold addition quantities.

According to the embodiment, the speed calculation unit 411 calculates a moving speed of the capsule endoscope 10 based on the chronological change of the coordinates of the estimated position of the capsule endoscope 10 estimated by the position estimation unit 213. Then, the distance threshold modification unit 311 sets a distance threshold addition quantity based on the moving speed of the capsule endoscope 10 calculated by the speed calculation unit 411 and the distance threshold addition quantity table stored in the storage unit 214. In addition, the distance threshold modification unit 311 sets a value obtained by adding the set distance threshold addition quantity to each value of the data item "distance threshold" of the position database as a distance threshold.

According to the embodiment, the frame rate-setting unit 215 determines the value of the data item "frame rate" stored as the set with the value of the data item "coordinates" as a frame rate of the imaging unit 101 of the capsule endoscope 10 when the distance between the position of the capsule endoscope 10 estimated by the position estimation unit 213 and the position shown by the value of the data item "coordinates" stored in the position database is less than the distance threshold set by the distance threshold modification unit 311. The wireless communication unit 211 then transmits the information showing the determined frame rate of the imaging unit 101 of the capsule endoscope 10 to the capsule endoscope 10 via the antenna elements 221 of the antenna section 22.

According to the embodiment, the imaging unit 101 of the capsule endoscope 10 performs imaging at the frame rate designated by the information showing the frame rate transmitted from the extracorporeal terminal 40. Accordingly, since the frame rate of the imaging unit 101 can be modified according to the position of the capsule endoscope 10, excessive consumption of the battery of the capsule endoscope 10 can be reduced. Additionally, in addition to a positional variation according to movement of internal organs in the body cavity or lumen thicknesses of the internal organs, since the distance threshold addition quantity is modified according to the moving speed of the capsule endoscope 10, modification of a more appropriate frame rate can be provided to the capsule endoscope 10.

### (Sixth embodiment)

Next, a sixth embodiment of the present invention will be described with reference to the accompanying drawings. A capsule endoscope system according to the embodiment includes the capsule endoscope 10 and the extracorporeal terminal 50, as in the fourth embodiment. In addition, the capsule endoscope 10 according to the embodiment is similar to the capsule endoscope 10 according to the fourth embodiment. In addition, the extracorporeal terminal 50 according to the embodiment is similar to the extracorporeal terminal 50 according to the fourth embodiment. A configuration of the embodiment differs from the fourth embodiment in that the storage unit 214 of the extracorporeal terminal 50 stores a distance threshold addition table and the position database has a different configuration. The configuration of the position database of the embodiment is similar to that of the position database according to the second embodiment.

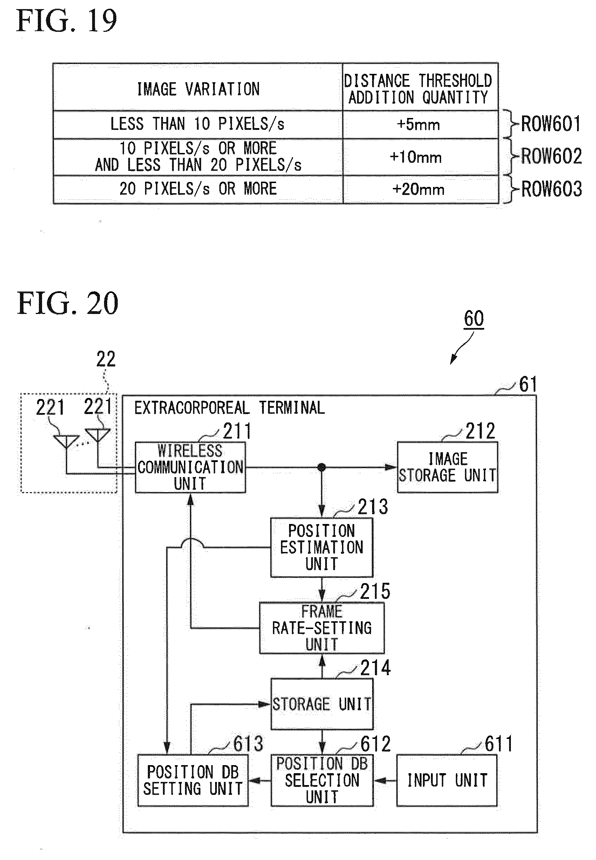

Next, the distance threshold addition table will be described. Fig. 19 is a schematic view showing a data structure of the distance threshold addition table of the embodiment. The distance threshold addition table has data items of "image variation" and "distance threshold addition quantity" and stores data of the data items in association with each row. The data item "image variation" stores an image variation. The data item "distance threshold addition quantity" stores a distance threshold addition quantity.

In the example shown, a value stored in the data item "image variation" in row 601 is "less than 10 pixels/s" and a value stored in the data item "distance threshold addition quantity" is "+5 mm." Thus, when the image variation is less than 10 pixels per second, 5 mm is added to the distance threshold.

In addition, in the example shown, a value stored in the data item "image variation" in row 602 is "10 pixels/s or more and less than 20 pixels/s" and a value stored in the data item "distance threshold addition quantity" is "+10 mm." Thus, when the image variation is 10 pixels or more and less than 20 pixels per second, 10 mm is added to the distance threshold.

In addition, in the example shown, a value stored in the data item "image variation" in row 603 is "20 pixels/s or more" and a value stored in the data item "distance threshold addition quantity" is "+20 mm." Thus, when the image variation is 20 pixels or more per second, 20 mm is added to the distance threshold.

In the example shown in Fig. 19, although three sets of values of the data item "image variation" and the values of the data item "distance threshold addition quantity" are stored, the example is not limited thereto. In addition, before the patient actually swallows the capsule endoscope 10, contents of the distance threshold addition table are set.

Next, a method of setting the distance threshold addition quantity using the distance threshold modification unit 311 will be described. The distance threshold modification unit 311 acquires the image variation calculated by the image variation calculation unit 511 and sets the value among the distance threshold addition quantities stored in the distance threshold addition table in association with the acquired image variation as a distance threshold addition quantity. For example, the distance threshold modification unit 311 sets the distance threshold addition quantity to 5 mm when the image variation calculated by the image variation calculation unit 511 is 3 pixels per second.

Next, a method of setting the distance threshold using the distance threshold modification unit 311 will be described. The distance threshold modification unit 311 sets a value obtained by adding the set distance threshold addition quantity to the value of the data item "distance threshold" of the position database as a distance threshold. For example, when the distance threshold addition quantity is 5 mm, the distance threshold modification unit 311 sets a distance threshold in the case of using the value (100, 180, 250) of the data item "coordinates" to 20 mm + 5 mm = 25 mm. The distance threshold modification unit 311 sets a distance threshold in the case of using the value (100, 100, 200) of the data item "coordinates" to 30 mm + 5 mm = 35 mm. The distance threshold modification unit 311 sets a distance threshold in the case of using the value (100, 220, 50) of the data item "coordinates" to 20 mm + 5 mm = 25 mm.

Next, a method of determining the frame rate of the imaging unit 101 of the capsule endoscope 10 using the frame rate-setting unit 215 will be described. The frame rate-setting unit 215 determines the frame rate of the imaging unit 101 of the capsule endoscope 10 as a frame rate stored in association with the coordinates when the distance between the position of the capsule endoscope 10 estimated by the position estimation unit 213 and the position shown by the coordinates stored in the position database is less than the distance threshold set by the distance threshold modification unit 311.

Next, an operation of the capsule endoscope 10 will be described. The operation of the capsule endoscope 10 is similar to that of the capsule endoscope 10 of the first embodiment.

Next, an operation of the extracorporeal terminal 50 will be described. The extracorporeal terminal 50 stores the image data transmitted from the capsule endoscope 10 in the image storage unit 212. In addition, the extracorporeal terminal 50 determines the frame rate of the imaging unit 101 of the capsule endoscope 10 and transmits the information showing the determined frame rate to the capsule endoscope 10.

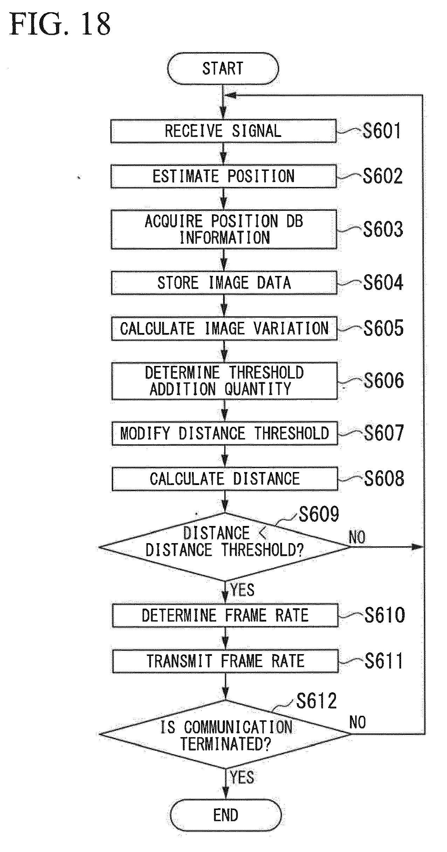

Fig. 18 is a flow chart showing an operational sequence in which the extracorporeal terminal 50 of the embodiment determines the frame rate of the capsule endoscope 10 and transmits the information showing the determined frame rate to the capsule endoscope 10.

Processing of step S601 to step S603 is similar to the processing of step S101 to step S103 of the first embodiment.
(Step S604) The image storage unit 212 stores the image data received from the capsule endoscope 10 by the wireless communication unit 211. The operation then proceeds to step S605. Processing of step S604 is referred to as an image storage step.
(Step S605) The image variation calculation unit 511 calculates an image variation using the image data stored in the image storage unit 212. The operation then proceeds to step S606. The processing of step S605 is referred to as an image variation calculation step.
(Step S606) The distance threshold modification unit 311 sets a distance threshold addition quantity based on the image variation calculated by the image variation calculation unit 511 in the image variation calculation step of step S605 and the distance threshold addition table stored in the storage unit 214. The operation then proceeds to step S607. The processing of step S606 is referred to as a distance threshold addition quantity determination step.
(Step S607) The distance threshold modification unit 311 sets a value obtained by adding the distance threshold addition quantity set in the distance threshold addition quantity determination step of step S606 to each value of the data item "distance threshold" of the position database as a distance threshold. The operation then proceeds to step S608. The processing of step S607 is referred to as a distance threshold modification step.
(Step S608) The frame rate-setting unit 215 calculates a distance between the two points of the position shown by the coordinate information of the capsule endoscope 10 output from the position estimation unit 213 in the position estimation step of step S602 and the position shown by the value of the data item "coordinates" read in the position database information acquisition step of step S603 for each value of the data item "coordinates." The operation then proceeds to step S609. The processing of step S608 is referred to as a distance calculation step.
(Step S609) The frame rate-setting unit 215 determines whether any one of the distances between the two points calculated in the distance calculation step of step S608 is less than the distance threshold set by the distance threshold modification unit 311 in the distance threshold modification step of step S607. When the frame rate-setting unit 215 determines that a distance among the distances between the two points calculated in the distance calculation step of step S608 is less than the distance threshold set by the distance threshold modification unit 311 in the distance threshold modification step of step S607, the operation proceeds to step S610, and in the other cases, the operation returns to the processing of step S601. The processing of step S609 is referred to as a threshold comparison and decision step.

Processing of step S610 to step S612 is similar to the processing of step S106 to step S108 of the first embodiment.

As described above, according to the embodiment, the position estimation unit 213 of the extracorporeal terminal 50 performs position detection of the capsule endoscope 10 using the signal levels received by the plurality of antenna elements 221 included in the antenna section 22. In addition, the position database stored in the storage unit 214 prestores sets of values of the data item "coordinates" showing reference positions of the capsule endoscope 10, values of the data item "frame rate" showing frame rates corresponding to the position of the capsule endoscope 10 and values of the data item "distance threshold" showing distance thresholds. In addition, the distance threshold addition table stored in the storage unit 214 prestores sets of values of the data item "image variation" showing image variations and values of the data item "distance threshold addition quantity" showing distance threshold addition quantities.

According to the embodiment, the image variation calculation unit 511 calculates an image variation using the image data stored in the image storage unit 212. Then, the distance threshold modification unit 311 sets a distance threshold addition quantity based on the image variation calculated by the image variation calculation unit 511 and the distance threshold addition quantity table stored in the storage unit 214. In addition, the distance threshold modification unit 311 sets a value obtained by adding the set distance threshold addition quantity to each value of the data item "distance threshold" of the position database as a distance threshold.

According to the embodiment, the frame rate-setting unit 215 determines the value of the data item "frame rate" stored as a set with the value of the data item "coordinates" as a frame rate of the imaging unit 101 of the capsule endoscope 10 when the distance between the position of the capsule endoscope 10 estimated by the position estimation unit 213 and the position shown by the values of the data item "coordinates" is less than the distance threshold set by the distance threshold modification unit 311. The wireless communication unit 211 then transmits the information showing the determined frame rate of the imaging unit 101 of the capsule endoscope 10 to the capsule endoscope 10 via the antenna elements 221 of the antenna section 22.

According to the embodiment, the imaging unit 101 of the capsule endoscope 10 performs imaging at the frame rate designated by the information showing the frame rate transmitted from the extracorporeal terminal 50. Accordingly, since the frame rate of the imaging unit 101 can be modified according to the position of the capsule endoscope 10, excessive consumption of a battery of the capsule endoscope 10 can be reduced. Additionally, in addition to a positional variation according movement of internal organs in the body cavity and lumen thicknesses of the internal organs, since the distance threshold addition quantity is modified according to the image variation calculated from the image data, modification of the frame rate can be more reliably instructed to the capsule endoscope 10.

### (Seventh embodiment)

Next, a seventh embodiment of the present invention will be described with reference to the accompanying drawings. A capsule endoscope system according to the embodiment includes a capsule endoscope and an extracorporeal terminal, as in the first embodiment. In addition, the capsule endoscope according to the embodiment is similar to the capsule endoscope according to the first embodiment. The embodiment differs from the first embodiment in that the extracorporeal terminal according to the embodiment includes an input unit, a position database selection unit and a position database-setting unit, and may easily update the value of the data item "coordinates" of the position database.

Fig. 20 is a block diagram showing a configuration of an extracorporeal terminal 60 of the embodiment. In an example shown, the extracorporeal terminal 60 includes a main body section 61 and the antenna section 22. A configuration of the antenna section 22 is similar to that of the antenna section 22 of the first embodiment. The main body section 61 includes the wireless communication unit 211, the image storage unit 212, the position estimation unit 213, the storage unit 214, the frame rate-setting unit 215, an input unit 611, a position database selection unit 612 and a position database-setting unit 613. The wireless communication unit 211, the image storage unit 212, the position estimation unit 213, the storage unit 214 and the frame rate-setting unit 215 are similar to the respective parts of the first embodiment.

The input unit 611 is constituted by an input device such as a touch panel or the like and receives input of input manipulation information. The position database selection unit 612 selects one set among sets (data sets) of values of the data item "coordinates" stored in the position database and values of the data item "frame rate" according to the input manipulation information obtained by receiving the input in the input unit 611. The position database-setting unit 613 sets the coordinates showing the position of the capsule endoscope 10 estimated by the position estimation unit 213 to the value of the data item "coordinates" of the set selected by the position database selection unit 612.

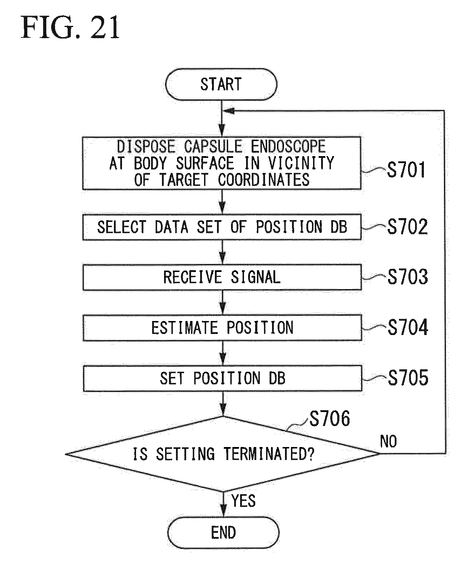

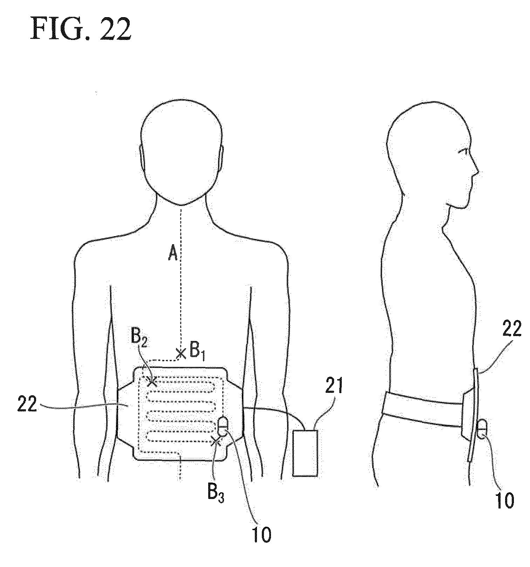

Next, a method of updating the values of the data item "coordinates" of the position database will be described. Fig. 21 is a flow chart showing an operational sequence in which the extracorporeal terminal 60 of the embodiment updates the values of the data item "coordinates" of the position database.
(Step S701) An operator such as a doctor, a patient, or the like, disposes an activated capsule endoscope 10 at a body surface in the vicinity of a position registered in the position database. The operation then proceeds to step S702. Fig. 22 is a schematic view showing a relationship between the coordinates of the embodiment and the human body. In the example shown, the example in which the value of the data item "coordinates" of the position database are coordinates showing a body surface B3, and the capsule endoscope 10 is disposed at the body surface B3.
   Hereinafter, a further description of Fig. 21 will be given.
(Step S702) The operator such as the doctor, the patient, or the like, manipulates the input unit 611, and performs the input that designates an updated value among the values of the data item "coordinates" of the position database. The position database selection unit 612 selects the updated value among the values of the data item "coordinates" of the position database based on the input received in the input unit 611. The operation then proceeds to step S703. The processing of step S702 is referred to as a position database selection step.
(Step S703) The plurality of antenna elements 221 receive signals (modulated signals or non-modulated carrier waves) transmitted from the capsule endoscope 10. Next, the wireless communication unit 211 measures signal levels (received signal intensities) of the signals received by the plurality of antenna elements 221. The operation then proceeds to step S704. The processing of step S703 is referred to as a signal-receiving step.
(Step S704) The position estimation unit 213 estimates the position of the capsule endoscope 10 using the signal levels (received signal intensities) of the signals received by the plurality of antenna elements 221 measured by the wireless communication unit 211 in the processing of step S703. The operation then proceeds to step S705. The processing of step S704 is referred to as a position estimation step.
(Step S705) The position database-setting unit 613 sets the coordinates showing the position of the capsule endoscope 10 estimated by the position estimation unit 213 in the position estimation step of step S704 to the value of the data item "coordinates" of the set selected by the position database selection unit 612 in the position database selection step of step S702. In actuality, due to a separation in distance between the body surface and the position in the body cavity, the operator such as the doctor, the patient, or the like may offset a designated value input into the input unit 611 or a fixed value to be set to the value of the data item "coordinates" of the position database. The operation then proceeds to step S706. The processing of step S705 is referred to as a position database-setting step.
(Step S706) The operator such as the doctor, the patient, or the like, inputs a predetermined termination manipulation into the input unit 611 when the processing is terminated. The input unit 611 terminates the processing when the predetermined termination manipulation (for example, push-down of a registration modification end button) is detected, and in the other cases, the operation returns to the processing of step S701.

The imaging processing of the capsule endoscope 10 or the setting processing of the frame rate of the capsule endoscope 10 may use any one of the processes described in the above-mentioned first embodiment to sixth embodiment.

By the above-mentioned configuration and operation, according to the embodiment, for example, the extracorporeal terminal 60 can receive signals from the capsule endoscope 10 adhered to the body surface and update the position database according to instruction of the doctor before the patient swallows the capsule endoscope 10. As a result, in consideration of an individual difference such as a body shape, an attachment condition of the antenna section 22, or the like, modification of the frame rate can be performed.

Hereinabove, although the first embodiment to the seventh embodiment of the present invention have been described in detail with reference to the accompanying drawings, a specific configuration is not limited to the embodiments but may include designs or the like without departing from the spirit of the present invention.

All or some of the functions of the respective parts included in the capsule endoscope 10 and the extracorporeal terminals 20, 30, 40, 50 and 60 according to the above-mentioned first embodiment to seventh embodiment may be realized by recording programs for realizing these functions on a computer-readable recording medium, reading the program recorded on the recording medium using a computer system, and executing the programs using the computer system. Here, the "computer system" includes an OS, hardware such as peripheral devices, or the like.

In addition, the "computer-readable recording medium" is a portable recording medium such as a flexible disk, a magneto-optical disk, a ROM, a CD-ROM, or the like, and a storage unit such as a hard disk or the like built into the computer system. Further, the "computer-readable recording medium" may include a medium configured to dynamically retain the program for a short time like a communication line when the program is transmitted via a network such as the Internet or the like or a communication line such as a telephone line or the like, and a medium configured to temporarily retain the program like a volatile memory in the computer system that becomes a server or a client in this case. In addition, the above-mentioned program may be used to realize some of the above-mentioned functions, or may realize the above-mentioned functions in combination with a prerecorded program recorded in the computer system.

Hereinabove, although the exemplary embodiments of the present invention have been described, the present invention is not limited to these embodiments. Addition, omission, substitution and the other modification of the components may be made without departing from the spirit of the present invention. The present invention is not limited to the above-mentioned description but is limited only by the accompanying claims.

### [Industrial Applicability]

The above-mentioned embodiments can provide an extracorporeal terminal, a capsule endoscope system, a capsule-endoscope control method and a program that are capable of setting the frame rate while suppressing an increase in electric power consumption of the capsule endoscope.

### [Reference Signs List]

- 1: capsule endoscope system
- 10: capsule endoscope
- 20, 30, 40, 50, 60: extracorporeal terminal
- 21, 31, 41, 51, 61: main body section
- 22: antenna section
- 101: imaging unit
- 102: wireless communication unit
- 103: control unit
- 211: wireless communication unit
- 212: image storage unit
- 213: position estimation unit
- 214: storage unit
- 215: frame rate-setting unit
- 221: antenna element
- 311: distance threshold modification unit
- 411: speed calculation unit
- 511: image variation calculation unit
- 611: input unit
- 612: position database selection unit
- 613: position database-setting unit

## Claims

1. An extracorporeal terminal, comprising:
a wireless communication unit including a plurality of antenna elements, and configured to transmit and receive a signal with a capsule endoscope that is capable of being set with a frame rate;
a storage unit configured to retain a position database showing relationships between coordinates and the frame rate;
a position estimation unit configured to estimate a position of the capsule endoscope using a signal level of the signal received by the wireless communication unit; and
a frame rate-setting unit configured to set the frame rate corresponding to the coordinates when a distance between a position shown by the coordinates included in the position database retained in the storage unit and a position of the capsule endoscope estimated by the position estimation unit is less than a distance threshold that is preset,
wherein the wireless communication unit transmits a signal showing the frame rate set by the frame rate-setting unit to the capsule endoscope.

2. The extracorporeal terminal according to Claim 1, further comprising a distance threshold modification unit configured to modify the distance threshold.

3. The extracorporeal terminal according to Claim 2, further comprising a speed calculation unit configured to calculate a moving speed of the capsule endoscope based on an estimation result of the position estimation unit,
wherein the distance threshold modification unit sets a value of the distance threshold to a large value when the moving speed calculated by the speed calculation unit is larger than a first speed, and sets a value of the distance threshold to a small value when the moving speed is smaller than a second speed.

4. The extracorporeal terminal according to Claim 2, further comprising:
an image storage unit configured to store image data received by the wireless communication unit and captured by the capsule endoscope; and
an image variation calculation unit configured to calculate an image variation serving as a chronological differential quantity of the image data stored in the image storage unit,
wherein the distance threshold modification unit sets a value of the distance threshold to a large value when the image variation calculated by the image variation calculation unit is larger than a first variation, and sets a value of the distance threshold to a small value when the image variation is smaller than a second variation.

5. The extracorporeal terminal according to Claim 2, further comprising a speed calculation unit configured to calculate a moving speed of the capsule endoscope based on an estimation result of the position estimation unit,
wherein the distance threshold modification unit sets a value obtained by adding a preset threshold addition quantity to the distance threshold as a distance threshold when the moving speed calculated by the speed calculation unit is larger than a predetermined speed.

6. The extracorporeal terminal according to Claim 2, further comprising:
an image storage unit configured to store image data received by the wireless communication unit and captured by the capsule endoscope; and
an image variation calculation unit configured to calculate an image variation serving as a chronological differential quantity of the image data stored in the image storage unit,
wherein the distance threshold modification unit sets a value obtained by adding a preset threshold addition quantity to the distance threshold as a distance threshold when the image variation calculated by the image variation calculation unit is larger than a predetermined variation.

7. The extracorporeal terminal according to Claim 1, further comprising:
a position database selection unit configured to select one of the relationships between the coordinates and the frame rates included in the position database retained in the storage unit; and
a position database-setting unit configured to set the coordinates selected by the position database selection unit to coordinates showing the position estimated by the position estimation unit.

8. A capsule endoscope system comprising a capsule endoscope and an extracorporeal terminal,
the capsule endoscope including:
a capsule communication unit configured to transmit and receive a signal with the extracorporeal terminal; and
an imaging unit configured to capture an image based on a signal showing a frame rate transmitted from the extracorporeal terminal,
the extracorporeal terminal including:
a wireless communication unit including a plurality of antenna elements and configured to transmit and receive the signal with the capsule endoscope;
a storage unit configured to retain a position database showing relationships between coordinates and frame rates;
a position estimation unit configured to estimate a position of the capsule endoscope using a signal level of the signal received by the wireless communication unit; and
a frame rate-setting unit configured to set the frame rate corresponding to the coordinates when a distance between a position shown by the coordinates included in the position database retained in the storage unit and a position of the capsule endoscope estimated by the position estimation unit is less than a preset distance threshold,
wherein the wireless communication unit transmits the signal showing the frame rate set by the frame rate-setting unit to the capsule endoscope.

9. A capsule-endoscope control method, comprising:
a wireless communication step in which a wireless communication unit including a plurality of antenna elements transmits and receives a signal with a capsule endoscope that is capable of being set with a frame rate;
a position estimation step of estimating a position of the capsule endoscope using a signal level of the signal received by the wireless communication unit; and
a frame rate-setting step of setting a frame rate corresponding to coordinates when a distance between a position shown by coordinates included in a position database showing a relationship between coordinates and frame rates retained in a storage unit and the position of the capsule endoscope estimated in the position estimation step is less than a preset distance threshold,
wherein, in the wireless communication step, a signal showing the frame rate set in the frame rate-setting step is transmitted to the capsule endoscope.

10. A program, comprising:
a wireless communication step in which a wireless communication unit including a plurality of antenna elements transmits and receives a signal with a capsule endoscope that is capable of being set with a frame rate;
a position estimation step of estimating a position of the capsule endoscope using a signal level of the signal received by the wireless communication unit; and
a frame rate-setting step of setting a frame rate corresponding to coordinates when a distance between a position shown by coordinates included in a position database showing a relationship between coordinates and frame rates retained in a storage unit and the position of the capsule endoscope estimated in the position estimation step is less than a preset distance threshold,
wherein, in the wireless communication step, a process of transmitting a signal showing the frame rate set in the frame rate-setting step to the capsule endoscope is executed in a computer.
